(19) Europäisches Patentamt
European Patent Office
Office européen des brevets

(11) Numéro de publication: **0 469 983 B1**

(12) # FASCICULE DE BREVET EUROPEEN

(45) Date de publication de fascicule du brevet: **22.02.95**

(51) Int. Cl.[6]: **C07C 409/24**, C08B 37/00, A01N 37/16, C11D 3/39, A61K 31/19, A61K 7/00

(21) Numéro de dépôt: **91402121.7**

(22) Date de dépôt: **30.07.91**

(54) **Clathrates de peroxyacides, leur préparation et leurs utilisations.**

(30) Priorité: **30.07.90 FR 9009677**

(43) Date de publication de la demande:
**05.02.92 Bulletin 92/06**

(45) Mention de la délivrance du brevet:
**22.02.95 Bulletin 95/08**

(84) Etats contractants désignés:
**AT BE CH DE DK ES FR GB GR IT LI LU NL SE**

(56) Documents cités:
**EP-A- 0 411 951**
**FR-A- 2 596 617**

**BULLETIN OF THE CHEMICAL SOCIETY OF JAPAN, vol. 43, no. 6, 1970, Tokyo, JP, page 1910; Y. MATSUI et al, "Stabilization of hydroperoxides by means of the formation of inclusion compounds with beta-cyclodextrin"**

(73) Titulaire: **CHEMOXAL S.A.**
**75 quai d'Orsay**
**F-75007 Paris (FR)**

(72) Inventeur: **Granger, Michel**
**24 place Mathias**
**F-71100 Chalons sur Saone (FR)**
Inventeur: **Dupont, Michel**
**Rue de l'Eglise,**
**Chaudenay**
**F-71150 Chagny (FR)**
Inventeur: **Ledon, Henry**
**1bis rue de l'Assemblée Nationale**
**F-78000 Versailles (FR)**

(74) Mandataire: **Caen, Thierry Alain et al**
**L'Air Liquide**
**Société anonyme pour l'étude et l'exploitation**
**des procédés Georges Claude**
**75, quai d'Orsay**
**F-75321 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention a pour objet de nouveaux dérivés de peroxyacides leur procédé de préparation et leurs utilisations.

On sait que les peroxyacides sont des composés difficiles à manipuler. A l'état cristallisé, ils sont en effet très souvent instables. Quant aux peroxyacides liquides, leur caractère explosif rend difficile, voire impossible leur purification.

Pour résoudre le problème résultant de leur instabilité et afin de pouvoir disposer aisément de ces composés, les inventeurs ont étudié la possibilité de les associer à d'autres molécules.

Ces travaux les ont conduits à étudier et à développer une nouvelle gamme de composés.

L'invention a donc pour but de fournir de nouveaux dérivés de peroxyacides sous une forme stable durant une longue période de stockage, permettant de disposer aisément des peroxyacides au moment de l'emploi.

L'invention vise également un procédé d'obtention de ces dérivés de mise en oeuvre aisée ainsi que leurs applications, en particulier dans le domaine de la désinfection, du blanchiment et en synthèse organique.

Les nouveaux dérivés de peroxyacides de l'invention sont caractérisés en ce qu'il s'agit de composés d'inclusion, ou clathrates, consistant en un peroxyacide inclus dans une molécule creuse capable de jouer le rôle de structure d'accueil vis-à-vis dudit peroxyacide.

L'association entre les peroxyacides et la molécule hôte est essentiellement basée sur les multiples liaisons de Van der Waals intervenant entre les deux composés. Dès la remise en solution du clathrate ou par chauffage, la substance insérée est libérée et donc disponible pour l'emploi.

Les molécules servant de structure d'accueil sont choisies parmi les composés inertes, dans les conditions utilisées pour leur préparation, vis-à-vis des peroxyacides, formant une cavité hydrophobe de forme et de dimensions appropriées pour contenir au moins une molécule de peroxyacide, et capable de retenir au sein de sa cavité la ou les molécules de peroxyacide.

D'une manière générale, des molécules hôte de ce type comprennent notamment des cavitands tels qu'évoqués par Peterson dans Science News, vol. 132, 90-93, 1987 et Milgrom dans New Scientist, 61-64, 1988, ou encore des cyclophanes (voir chapitre 11, p. 629 et suivantes, de Odashima et al dans Academic Press, 1983, ed. par P.M. Keehn et S.M. Rosenfeld).

Dans un mode préféré de réalisation de l'invention, la molécule réceptrice présente la structure d'une cyclodextrine.

On sait que les cyclodextrines (CD) sont des oligoglucosides cycliques obtenus par dégradation enzymatique de l'amidon. Elles répondent à la formule $(C_6H_{10}O_5)_n$.

On a déjà décrit des dérivés d'inclusion dans les cyclodextrines (voir cyclodextrins and their inclusion complexes de J. Szejtli, Akademiai kiado, Budapest, 1982).

Parmi ces dérivés, on a envisagé l'inclusion des acides organiques et de certains hydroperoxydes organiques essentiellement aux fins d'amélioration de leur stabilité thermique et de diminution de leur tension de vapeur.

Cependant, les mêmes techniques appliquées à la micro-encapsulation de $H_2O_2$ n'ont pas permis d'isoler des clathrates cristallisés.

Il était donc totalement inattendu d'inclure conformément à l'invention un peroxyacide dans une structure d'accueil du type cyclodextrine.

Des cyclodextrines spécialement appropriées selon l'invention comprennent l'alpha-cyclodextrine (ou cyclohexamylose), la bêta-cyclodextrine (ou cyclo-heptamylose) ou encore la gamma-cyclodextrine (ou cyclooctamylose).

Les cyclodextrines utilisées sont le cas échéant substituées lorsqu'on souhaite leur conférer des propriétés spécifiques. A titre d'exemple, on citera les substitutions par des groupes alkyle, maltosyle ou hydroxypropyle, et ceux décrits dans l'article de J. Szejtli, précédemment cité.

Compte tenu du procédé d'obtention utilisé, les cyclodextrines sont le plus généralement hydratées. Cette teneur résiduelle en eau joue un rôle avantageux sur la conservation, favorisant une libération lente du peroxyacide à l'humidité de l'air.

Le diamètre de la cavité est de 5 à 6 Angstroms pour l'alpha-cyclodextrine et sa profondeur de 7 à 8 Angstroms.

Pour inclure une molécule de peroxyacide, cet ordre de grandeur des dimensions d'une structure d'accueil apparaît avantageux.

Comme peroxyacides utilisables pour l'encapsulation dans les cyclodextrines, on citera :

- les mono-peroxyacides de structure $R_1$-$CO_3H$, avec $R_1$ représentant un groupe alkyle, aryle, cycloalkyle ;
- les diperoxyacides de structure $HO_3C$-$R_2$-$CO_3H$, avec $R_2$ représentant une simple liaison ou un groupe alkylène, arylène, cycloalkylène.

De manière préférée, le terme alkyle ou alkylène dans les significations ci-dessus correspond à des radicaux comprenant de 1 à 12 atomes de carbone ; le groupe aryle est de préférence un phényle et le groupe arylène, un phénylène.

Les substituants $R_1$ et $R_2$ sont le cas échéant substitués par des groupements fonctionnels, en particulier par une ou plusieurs fonctions carboxyliques, sous forme ester ou amide, ou de sels, notamment de sels alcalins, alcalino-terreux, d'ammonium ou de phosphonium, et/ou par un ou plusieurs groupes alkyle, alkoxy, aryle, le cas échéant à un ou plusieurs cycles, amino, alkylamino, acylamino, acyle, nitrile, nitro, trifluorométhyle, sulfonyle, et/ou un ou plusieurs atomes d'halogène.

Selon l'une des dispositions de l'invention, les peroxyacides sont en mélange avec les acides organiques correspondants selon des teneurs pouvant varier dans un large intervalle allant de quelques pourcents jusqu'à environ 90 % en poids, de préférence de 10 à 40 % environ.

Selon une autre disposition, les peroxyacides solides à la température ambiante sont en général obtenus sous forme de mélanges riches en peroxyacides et ne contiennent pas beaucoup d'acide non peroxydé. La teneur en peroxyacide est ainsi le plus souvent d'au moins environ 80 % en poids.

Les peroxyacides en solution, pour des raisons de sécurité, ne renferment généralement pas plus de 40 à 50 % environ de peroxyacide, ce dernier étant en mélange avec l'acide organique correspondant et le peroxyde d'hydrogène, respectivement à raison d'environ 10 à 20 % et 20 à 25 %, en poids, avec éventuellement un catalyseur comprenant un acide fort tel que $H_2SO_4$ ou une résine permettant d'obtenir rapidement l'équilibre du mélange recherché.

L'invention concerne particulièrement les clathrates de l'acide peracétique et ceux de l'acide perpropionique. Ces composés se présentent sous forme de poudres cristallines, blanches, inodores.

Selon un aspect de grand intérêt, leur teneur en peroxyacide reste stable au stockage à la température ambiante. Leur stabilité est au moins aussi bonne que celle d'une solution aqueuse, et le plus généralement nettement supérieure, comme par exemple avec l'acide perpropionique.

On mesurera également l'intérêt de ces clathrates qui permettent une manipulation à la main, sans précaution particulière et sans nuisance d'odeurs, de produits connus pour leur caractère corrosif, très souvent explosif, et leur odeur prononcée.

Le clathrate constitue également un moyen de stocker le peroxyacide en évitant toute pollution par un récipient les contenant.

L'invention s'applique aux peroxyacides aliphatiques, solides à la température ambiante, tels que les acides perazélaiques, percaprylique, perundécynélique, perlaurique, monoperoxysucciniques, monoperoxymaléique, monoperoxyglutarique ou diperoxysucciniques substitués par un groupement alkyle ou alkényle, tel que par exemple l'octyl-2-peroxybutanedioique-1,4, ou encore un diperoxyacide tel que l'acide diperoxydodécane dicarboxylique-1,12.

L'invention s'applique en outre avec avantage aux acides peroxybenzoiques répondant à la formule :

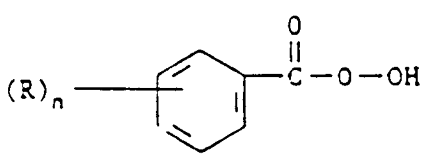

dans laquelle :

n est un nombre de 1 à 5 et R présente les significations données ci-dessus pour $R_1$ et $R_2$.

Comme acides de ce type, on citera l'acide peroxybenzoique, metachloroperoxybenzoique, p.tertiobutylperoxybenzoique, p-nitroperoxybenzoique, mono-perphtalique, ou amino-phtaloyl-peracétique,

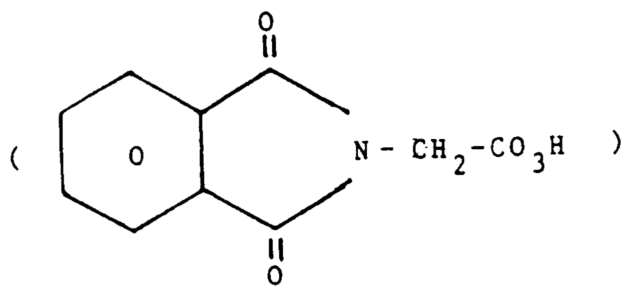

leurs sels et leurs dérivés, notamment les esters ou les amides tels que définis ci-dessus.

L'invention vise également un procédé d'obtention de dérivés d'inclusion de peroxyacides.

Ce procédé comprend l'addition de peroxyacide pur ou en mélange à un composé capable de jouer le rôle de structure d'accueil pour le peroxyacide et la récupération du clathrate formé.

La réaction est réalisée en l'absence de solvants ou, en variante, en milieu solvant hétérogène, ou encore, de préférence, en milieu solvant homogène.

Les peroxyacides solides à la température ambiante sont mis avantageusement en solution dans un solvant approprié, par exemple eau ou mélange eau ou solvant hydrosoluble tel qu'un alcool, et additionnés au composé d'accueil, lui-même avantageusement en solution.

Le clathrate précipite plus ou moins rapidement selon la température, la vitesse d'addition, les concentrations relatives. En jouant sur ces paramètres, il est possible de modifier la dimension des grains du précipité et de définir des conditions pour lesquelles la séparation du précipité sera facilitée.

Après séparation, plus spécialement par filtration ou essorage centrifuge, le précipité est séché par tous moyens adéquats connus de l'homme de l'art ; un moyen particulièrement efficace et bien adapté consiste à sécher par courant d'air, notamment à une température inférieure à 50 ° C environ.

Les peroxyacides solubles dans l'eau, notamment les alkylperoxyacides de poids moléculaire peu élevé, par exemple ceux comportant environ 1 à 6 atomes de carbone, sont préparés de préférence à partir des acides organique solubles dans l'eau correspondants et de peroxyde d'hydrogène selon la réaction équilibrée :

$$R\text{-}CO_2H + H_2O_2 \rightleftarrows R_1\text{-}CO_3H + H_2O \qquad (A)$$

Les inventeurs ont constaté que l'acide précurseur du peroxyacide peut donner un dérivé d'inclusion avec la CD alors que le peroxyde d'hydrogène n'est pas isolable sous forme cristallisée.

Pour obtenir un clathrate riche en peroxyacide et relativement pauvre en acide précurseur, on utilise avec avantage un mélange en équilibre tel que la réaction (A) soit le plus possible déplacée vers la droite ; pour atteindre le plus rapidement possible cet état d'équilibre, il est avantageux d'ajouter un catalyseur tel qu'un acide minéral fort à l'état liquide ou solide, comme l'acide sulfurique ou l'acide phosphorique ou une résine sulfonique, par exemple du type de celles connues sous la marque commerciale DOWEX$^{R}$ ; les limites de concentration du mélange seront aisément définies par l'homme de l'art en fonction de la sécurité et de la commodité des opérations.

Les solutions de peroxyacides ou les mélanges solides riches en peroxyacides peuvent contenir les stabilisants habituels des produits peroxydés, tel que par exemple l'acide dipicolinique.

Le rapport molaire peroxyacide/cyclodextrine varie en particulier de 0,5 à 3, un rendement satisfaisant étant obtenu avec un rapport de l'ordre de 1 à 2, plus spécialement voisin de 1. Dans le cas de mélanges du peroxyacide avec l'acide organique correspondant, le rapport molaire peroxyacide + acide organique/cyclodextrine est avantageusement de l'ordre de 1 à 2.

Dans le cas de l'alpha-CD, la précipitation du clathrate est obtenue d'une manière préférée par addition par exemple de la solution de peroxyacide à la solution aqueuse saturée d'alpha-CD en quantité équimoléculaire (équimolécularité sur la base de alpha-CD hydraté à $6H_2O$) ; le mélange est ensuite refroidi jusqu'au voisinage de 0 ° C environ ; le clathrate ainsi précipité est filtré et séché par tous moyens connus ; on obtient des résultats particulièrement intéressants en effectuant le séchage à température relativement basse inférieure ou égale à 35 ° C par un courant d'air traversant le lit de solide pulvérulent.

L'avantage de disposer des peroxyacides sous forme de composés d'inclusion facilite leur manipulation dans les diverses applications connues de ces produits et permet de les utiliser dans de nouvelles applications.

Leur stabilité à la température et au stockage, l'absence d'odeur, la totale sécurité dans leur manipulation, l'abaissement de leur tension de vapeur et le titre élevé en peroxyacide dont ils permettent de disposer au moment de l'emploi leur confère à cet égard un intérêt majeur.

Les clathrates de l'invention sont particulièrement utiles comme agents de blanchiment, en particulier dans les lessives liquides et solides.

Le pouvoir germicide des composés de l'invention les rend également précieux comme agents désinfectants. Ils peuvent être utilisés dans les applications classiques de désinfection, notamment de locaux et leurs atmosphères, de matériels, de conduits et canalisations, de réservoirs. On peut citer par exemple la désinfection des locaux d'élevage et de litières d'animaux, des serres de primeurs ou horticoles, des silos, des toilettes, des réserves et laboratoires des métiers de bouche, des transports en commun, de réseaux de distribution d'air conditionné, des infrastructures à usage collectif, comme les salles de sport, les piscines et les saunas, de réseaux d'eau potable, de gaines de distribution de fluides, de réseaux d'évacuation, de mobilier, des récipients de collecte et de transport du lait, des végétaux et des plantes, de conditionnements, de cuves, du matériel de concentration, comme des membranes d'ultrafiltration, notamment celui utilisé dans les industries agro-alimentaires, des fermenteurs, des locaux et du matériel dans les industries pharmaceutiques et cosmétiques, des eaux de process industriels, des boues de forages aquifères ou pétrolifères, de fluides de coupes.

Les composés de l'invention présentent un intérêt tout particulier pour la désinfection hospitalière ou médicale, d'une part pour les locaux et d'autre part, pour le matérial médico-chirurgical, notamment les instruments chirurgicaux et de dentisteries, les fibroscopes, les appareils d'hémodialyse, les seringues et, d'une façon plus générale, les objets pouvant présenter une contamination infectieuse ou virale, tels que le linge hospitalier, les pansements, les sanies.

Les composés de l'invention conviennent également comme désinfectant à usage d'hygiène domestique, pour la désinfection d'éviers, de salles de bains, de biberons, de lentilles de contact, de brosses à dents.

Ils peuvent aussi être utilisés comme agent d'hygiène corporelle, pour l'asepsie de la peau et des muqueuses, par exemple sous forme de savons ou de détergents liquides, notamment ceux destinés à l'asepsie des mains en bloc opératoire, comme agent d'hygiène buccale, par exemple sous forme de dentifrices ou de solutions pour bains de bouche.

Ils peuvent encore être utilisés comme agents cosmétiques, notamment en vue d'éliminer les comédons.

Enfin, les composés de l'invention peuvent être utilisés en tant que médicament, notamment sous forme de pansement antiseptique ou en tant qu'antiseptique en vue de lutter contre les infections du tube digestif, notamment les infections intestinales, de la peau, des muqueuses et des organes génitaux, en particulier la cavité génitale.

Les composés de l'invention s'avèrent particulièrement intéressants en raison de leur effet germicide, en particulier virucide, algicide, sporicide, bactéricide et fongicide.

Ils sont utilisés, éventuellement, en association avec un véhicule inerte, sous forme pulvérulente, le cas échéant propulsés par gaz vecteur, sous forme de granules, de comprimés ou de pastilles et ajoutés au milieu dans lequel on souhaite libérer le peroxyacide.

Des comprimés de clathrate sont par exemple obtenus par compression sèche.

A titre d'exemple, on prépare un clathrate d'acide peracétique par compression à sec de 30 mg de clathrate et de 70 mg d'un excipient comportant :

| | |
|---|---|
| - AVICEL$^R$ pH 102 | 78,7 % |
| - amidon | 20,0 % |
| - AEROSIL$^R$ 200 | 0,3 % |
| - stéarate de magnésium | 1,0 % |

Pour un comprimé de 100 mg comportant un composé selon l'invention, il est possible de faire varier la quantité d'excipient dans de grandes proportions, par exemple de 50 à 99 mg.

Les clathrates peuvent être également disposés dans un lieu à désinfecter, par exemple un conduit d'évacuation, et on peut les laisser se décomposer sous l'action de l'humidité, la molécule hôte se dissolvant et libérant le peroxyacide.

Les clathrates de l'invention sont également particulièrement intéressants en synthèse organique, dans toutes les réactions d'oxydation comportant la mise en oeuvre de peroxyacides.

Afin d'illustrer l'invention, on rapporte ci-après des exemples d'obtention de différents clathrates de peroxyacides.

**Exemple 1 : clathrate d'acide peracétique APA**

1.1. Préparation de la solution d'APA

On mélange 10 g d'acide acétique pur additionné éventuellement de 1,225 g/l d'acide dipicolinique et de 1 g de $H_2SO_4$ 98 % à 14 g d'$H_2O_2$ 70 % ; après 24 heures, on obtient un mélange titrant 37,5 % en acide peracétique, 10 % en acide acétique et 22,5 % en $H_2O_2$.

1.2. Précipitation/filtration - Séchage du clathrate

On ajoute 1,53 g de cette solution (soit 7,6 mmoles d'APA et 2,4 mmoles d'AA) à 80 g d'une solution aqueuse d'alpha-CD contenant 9,76 g (10 mmoles) (exprimé en produit anhydre) à 20 ° C ; après 40 mn d'agitation à 20 ° C, on refroidit jusqu'à 5 ° C en 4 H 30 ; on essore sur verre fritté et on sèche par courant d'air à 35 ° C : 20 mn avec une vitesse d'air de 0,17 m/sec puis 10 mn avec une vitesse d'air de 0,06 m/sec ; on obtient 5,1 g de clathrate titrant 4 % APA et 1,5 % d'acide acétique et environ 0,1 % $H_2O_2$ ; produit stocké à la température ordinaire en flacon de verre.

Les eaux-mères contenant de l'alpha-CD et éventuellement de l'APA peuvent être recyclées avec remises au titre.

**Exemple 2 : clathrate d'acide perpropionique APPROP**

2.1. Préparation du peroxyacide

A 2,48 g d'acide propionique pur contenant 1 % en poids d'acide sulfurique et éventuellement 0,12 % en poids d'acide dipicolinique, on ajoute 2,53 g d'$H_2O_2$ 70 % ; après 48 heures, cette solution titre 21,9 % $H_2O_2$, 34,8 % en peracide et 17 % en acide propionique.

2.2. Préparation du clathrate

Sur un pied de cuve contenant 24 g d'eau-mère d'une cristallisation précédente, on ajoute 0,74 g d'alpha-CD et 5,4 g d'eau puis 500 mg de cristaux de clathrate servant d'amorces puis, par pompe doseuse, on ajoute en une heure simultanément 83,9 g d'alpha-CD contenant 10,5 mmoles d'alpha-CD et 2,22 g de la solution d'acide perpropionique ; on laisse sous agitation encore 1 heure à 22 ° C.
Après filtration et séchage de façon analogue à l'exemple 1, on obtient 11,1 g de produit sec titrant 3,8 % en acide perpropionique et 2,9 % en acide propionique et 0,16 % en $H_2O_2$.

Stabilité au stockage des clathrates d'APA et d'acide perpropionique

Après 70 jours de stockage à la température ambiante, le clathrate obtenu dans l'exemple 1 a un titre équivalent à 94 % du titre initial.

Après 50 jours de stockage à la température ambiante, le clathrate d'acide perpropionique obtenu dans l'exemple 2 a un titre équivalent à 97 % du titre initial en peroxyacide.

La solution mère d'acide perpropionique conservée dans les mêmes conditions pendant 11 jours ne titre plus que 95 % du titre initial en peroxyacide.

**Exemple 3 : clathrate d'acide monoperoxysuccinique APS**

Le peroxyacide initial est un produit solide titrant 81 % APS, 6 % acide et 0,9 % en $H_2O_2$.

On prépare 4,2 g de solution aqueuse contenant 5,12 mmoles d'APS et 0,43 mmole d'acide et on l'ajoute rapidement à 136,5 g de solution bêta-cyclodextrine à 46 ° C contenant 5,5 mmoles de bêta-CD.

On refroidit lentement (6 heures) jusqu'à 10 ° C sous agitation.

Après filtration et séchage, on recueille 5,2 g de précipité contenant 2,3 % de peroxyacide.

**Exemple 4 : acide metachloroperbenzoique AMCPB**

4.1. Préparation du clathrate avec alpha-CD

L'acide métachloroperbenzoique initial est un solide titrant 87 % de peroxyacide et 12 % d'acide métachlorobenzoique.

On prépare 50 g d'une solution aqueuse contenant 6,57 mmoles d'alpha-CD (soit 6,39 g d'alpha-CD anhydre). On ajoute, en quelques secondes, 1,13 g d'AMCPB sous agitation à la température ambiante. On laisse sous agitation pendant 16 heures.

Après filtration et séchage, on obtient 4,1 g de précipité contenant 14 % de peroxyacide.

4.2. Préparation du clathrate avec bêta-CD

On prépare une solution à 40 °C contenant 91 g d'eau, 23 g d'éthanol et 6,49 g de bêta-CD. On prépare une seconde solution contenant 5 g d'éthanol et 0,95 g de AMCPB. Cette seconde solution est introduite dans la première en une minute environ, sous agitation, à 40 °C. Ce mélange est maintenu sous agitation pendant 17 h à température ambiante.

Après filtration et séchage, on obtient 6,5 g de précipité sec contenant 9,5 % de peroxyacide.

**Exemple 5 : acide paratertiobutylperbenzoique APTBPB**

Ce produit contient 90 % de peroxyacide et 10 % d'acide paratertiobutylbenzoique (APTBB).

5.1. Préparation du clathrate avec alpha-CD

On prépare 142 g de solution aqueuse contenant 12,5 mmoles d'alpha-CD (12,2 g alpha-CD anhydre) et 42 g d'éthanol. On prépare une seconde solution contenant 4 g d'éthanol et 2,4 g de APTBPB. La solution alcoolique de peracide est ajoutée à la première solution, sous agitation et à température ambiante. On laisse réagir sous agitation pendant 20 minutes.

Après filtration et séchage, on obtient 8,6 g de précipité contenant 7 % de peroxyacide.

5.2. Préparation du clathrate avec bêta-CD

On prépare une solution à 50 °C contenant 75 g d'eau, 25 g d'éthanol et 6,1 g de bêta-CD (soit 5,2 mmoles bêta-CD).

On prépare une seconde solution contenant 1,6 g d'éthanol et 1 g de peroxyacide.Cette solution est introduite lentement (en 3 mn) dans la première solution à 40 °C.

On laisse refroidir le mélange pendant 3 h 45 mn. Il se forme un précipité blanc. Après filtration et séchage, on obtient 5,6 g de précipité contenant 7,6 % de peroxyacide.

Etude du pouvoir désinfectant d'un clathrate d'acide peracétique (APA)

1. Des essais de désinfectation ont été réalisés selon le protocole de la norme AFNOR NFT 72150 en utilisant les produits suivants :
   - A : un clathrate d'APA renfermant 4 % d'APA (en poids),
   - B : à titre de comparaison, de l'APA à 35 % renfermant 9 % de peroxide d'hydrogène en mélange.
   - . Germe testé : Staphylococcus aureus, CNCM 53154
   - . Temps de contact : 5 minutes
   - . Chutes de germes en log : 6 log
   - . température : 20 °C

Résultats

CLATHRATE D'APA — numération INITIALE : 2,55 $10^7$ germes/ml

| Concentration produit % (masse/volume) | 0,025% | 0,05 | 0,0625 | 0,0750 |
|---|---|---|---|---|
| Concentration en ppm APA | 10 | 20 | 25 | 30 |
| Germe testé | | | | |
| Staphylococcus aureus | + | 0 chute 7,4 log | 0 | 0 |

Avec le produit B, la concentration minimale bactéricide est de 35 ppm d'APA selon le même protocole que les essais réalisés par les inventeurs, c'est-à-dire 0,01 % de produit.

La concentration minimale bactéricide de clathrate d'APA est de 20 ppm d'APA en 5 minutes. Elle est de 35 ppm d'APA avec le produit B, avec les mêmes conditions opératoires. Le clathrate a donc une meilleure activité germicide que le produit B dont l'efficacité est environ 2 moins forte que le premier.

2) D'autres essais de désinfection ont été réalisés selon le protocole de la norme AFNOR NFT 72150 en utilisant les produits suivants :

- A : un clathrate d'APA renfermant 4 % d'APA en poids ;
- C : une solution commerciale, à titre de comparaison, contenant 2,5 % d'acide peracétique et 18 % de peroxyde d'hydrogène en mélange ;
- temps de contact : 5 minutes ;
- chute minimale du titre bactérien recherchée : $10^5$/ml ;
- température : 20 °C

RESULTATS:

| Germe testé: Staphylococcus aureus CNCM 53 154 | | | | |
|---|---|---|---|---|
| Concentrations en produits | Concentrations en a.peracétique | Numération initiale | Numération finale | Chute en log10 |
| A = 0,04%<br>C = 0,25% | 16 ppm<br>62,5 ppm | 7,7 $10^8$ b/ml<br>7,7 $10^8$ b/ml | < 1 b/ml<br>250 b/ml | 8,89<br>6,49 |

| Germe testé: Pseudomonas aeruginosa IP A 22 | | | | |
|---|---|---|---|---|
| Concentrations en produits | Concentrations en a.peracétique | Numération initiale | Numération finale | Chute en log10 |
| A = 0,04%<br>C = 0,25% | 16<br>62,5 | 1,1 $10^9$ b/ml<br>1,1 $10^9$ b/ml | 4,7 $10^3$ b/ml<br>8,5 $10^3$ b/ml | 5,37<br>5,11 |

| Germe testé: Escherichia coli CNCM 54 127 | | | | |
|---|---|---|---|---|
| Concentrations en produits | Concentrations en a.peracétique | Numération initiale | Numération finale | Chute en log10 |
| A = 0,04%<br>C = 0,25% | 16<br>62,5 | 7,9 $10^8$ b/ml<br>7,9 $10^8$ b/ml | <1 b/ml<br>5,5 $10^3$ b/ml | 8,90<br>5,16 |

| Germe testé: Enterococcus faecium CIP 5855 | | | | |
|---|---|---|---|---|
| Concentrations en produits | Concentrations en a.peracétique | Numération initiale | Numération finale | Chute en log10 |
| A = 0,04%<br>C = 0,25% | 16<br>62,5 | 6,8 $10^8$ b/ml<br>6,8 $10^8$ b/ml | <1 b/ml<br>9,8 $10^2$ b/ml | 8,83<br>5,84 |

| Germe testé: Mycobacterium smegmatis CNCM 7 326 | | | | |
|---|---|---|---|---|
| Concentrations en produits | Concentrations en a.peracétique | Numération initiale | Numération finale | Chute en log10 |
| A = 0,4%<br>C = 1% | 160<br>250 | 3,7 $10^7$ b/ml<br>3,7 $10^7$ b/ml | 20 b/ml<br>190 b/ml | 6,27<br>5,29 |

Au vu des résultats ci-dessus, on peut constater que, quelque soit le germe testé, la chute du titre bactérien est toujours plus importante, voire beaucoup plus importante avec le produit A qu'avec le produit C, et ce, bien que le produit A présente une concentration en APA inférieure à celle du produit C, ce dernier étant par ailleurs associé avec un autre agent germicide, le peroxyde d'hydrogène.

## Revendications

**1.** Dérivés de peroxyacides, caractérisés en ce qu'il s'agit de composés d'inclusion, ou clathrates, consistant en un peroxyacide inclus dans une molécule creuse capable de jouer le rôle de structure d'accueil vis-à-vis dudit peroxyacide.

**2.** Dérivés de peroxyacides selon la revendication 1, caractérisés en ce que la molécule jouant le rôle de structure d'accueil présente la structure d'une cyclodextrine, notamment de l'alpha-cyclodextrine, la bêta-cyclodextrine ou de la gamma-cyclodextrine ou de dérivés de cyclodextrines.

**3.** Dérivés de peroxyacides selon la revendication 1 ou 2, caractérisés en ce que le peroxyacide est choisi parmi :

- les mono-peracides de structure $R_1$-$CO_3H$, avec $R_1$ représentant un groupe alkyle, aryle, cycloalkyle,
- les diperoxyacides de structure $HO_3C$-$R_2$-$CO_3H$,

avec $R_2$ représentant un groupe alkylène, arylène, cycloalkylène ; $R_1$ et $R_2$ étant le cas échéant substitués par des groupements fonctionnels, en particulier par une ou plusieurs fonctions carboxyliques, sous forme ester ou amide, ou de sels, notamment de sels alcalins, alcalino-terreux, d'ammonium ou de phosphonium, et/ou par un ou plusieurs groupes alkyle, alkoxy, aryle, le cas échéant à un ou plusieurs cycles, amino, alkylamino, acylamino, acyle, nitrile, nitro, trifluorométhyle, sulfonyle, et/ou un ou plusieurs atomes d'halogène.

**4.** Dérivés de peroxyacides selon la revendication 3, caractérisés en ce que les radicaux alkyle ou alkylène comprennent de 1 à 12 atomes de carbone, le radical aryle est un radical phényle et le radical arylène, un radical phénylène.

**5.** Dérivés de peroxyacides selon l'une quelconque des revendications 1 à 4, caractérisés en ce que les peroxyacides mis en oeuvre pour élaborer les clathrates sont en mélange avec les acides organiques correspondants selon des teneurs pouvant varier de quelques pourcents jusqu'à environ 90 % en moles, plus particulièrement de 10 à 40 % environ et que les peroxyacides solides à la température ambiante sont sous forme de mélanges dont la teneur en peroxyacide est d'au moins environ 80 % en poids.

**6.** Procédé de préparation de dérivés de peroxyacides selon la revendication 1, caractérisé en ce qu'il comprend l'addition de peroxyacide pur ou en mélange à un composé capable de jouer le rôle de structure d'accueil pour le peroxyacide et la récupération du clathrate formé.

**7.** Procédé selon la revendication 6, caractérisé en ce que les peroxyacides solides à la température ambiante sont mis avantageusement en solution dans un solvant approprié -par exemple eau ou mélange eau/solvant hydrosoluble tel qu'un alcool- et additionnés au composé d'accueil lui-même avantageusement en solution et que les peroxyacides solubles dans l'eau, notamment les alkylperoxyacides, par exemple ceux comportant de 1 à 6 atomes de carbone, se présentent sous forme de mélanges avec les acides carboxyliques correspondants et du peroxyde d'hydrogène, ces mélanges étant additionnés d'un catalyseur tel qu'un acide minéral fort comme l'acide sulfurique ou l'acide phosphorique ou d'une résine sulfonique.

**8.** Procédé selon la revendication 7, caractérisé en ce que la solution de peroxyacide comporte un agent stabilisant tel que l'acide dipicolinique.

**9.** Agents de blanchiment, caractérisés en ce qu'ils comportent au moins un dérivé de peroxyacide selon l'une quelconque des revendications 1 à 5, éventuellement en association avec un véhicule inerte.

**10.** Agents désinfectants, caractérisés en ce qu'ils comportent au moins un dérivé de peroxyacide selon l'une quelconque des revendications 1 à 5, éventuellement en association avec un véhicule inerte.

**11.** Agents cosmétiques, caractérisés en ce qu'ils comportent au moins un dérivé de peroxyacide selon l'une quelconque des revendications 1 à 5, éventuellement en association avec un véhicule inerte.

**12.** Agents d'hygiène corporelle, caractérisés en ce qu'ils comportent au moins un dérivé de peroxyacide selon l'une quelconque des revendications 1 à 5, éventuellement en association avec un véhicule inerte.

**13.** Médicaments comportant à titre de principe actif au moins un dérivé de peroxyacide selon l'une quelconque des revendications 1 à 5.

**14.** Utilisation des dérivés de peroxyacide selon l'une quelconque des revendications 1 à 5 pour l'obtention d'un médicament à activité antiseptique, notamment en vue de lutter contre les infections du tube digestif, de la peau, des muqueuses ou des organes génitaux.

**15.** Agents ou médicaments selon l'une quelconque des revendications 9 à 14, caractérisés en ce qu'ils se présentent sous forme de poudre, de comprimés ou de granulés, éventuellement en association avec un véhicule inerte.

**16.** Utilisation d'un dérivé de peroxyacide selon l'une des revendications 1 à 5 comme réactif en synthèse organique.

## Claims

1. Derivatives of peroxyacids, characterized in that inclusion compounds are concerned, or clathrates consisting of a peroxyacid included in a hollow molecule capable of acting as a structure receiving the said peroxyacid.

2. Derivatives of peroxyacids according to Claim 1, characterized in that the molecule acting as a receiving structure has the structure of a cyclodextrin, particularly alpha-cyclodextrin, beta-cyclodextrin or gamma-cyclodextrin or derivatives of cyclodextrins.

3. Derivatives of peroxyacids according to Claim 1 or 2, characterized in that the peroxyacid is selected from :
   - mono-peracids having the structure $R_1$-$CO_3H$, with $R_1$ representing an alkyl, aryl or cycloalkyl group.
   - diperoxyacids having the structure $HO_3C$-$R_2$-$CO_3H$,

   with $R_2$ representing an alkylene, arylene or cycloalkylene group; $R_1$ and $R_2$ being substituted where appropriate by functional groups, in particular by one or more carboxylic functional groups, in the form of an ester or amide, or salts, particularly alkali metal, alkaline earth, ammonium or phosphonium salts, and/or by one or more alkyl groups, alkoxy groups, aryl groups, as the case may be with one or more rings, amino groups, alkylamino groups, acylamino groups, acyl groups, nitrile groups, nitro groups, trifluoromethyl groups or sulphonyl groups and/or one or more halogen atoms.

4. Derivatives of peroxyacids according to Claim 3, characterized in that the alkyl or alkylene radicals comprise 1 to 12 carbon atoms, the aryl radical is a phenyl radical and the arylene radical is a phenylene radical.

5. Derivatives of peroxyacids according to any one of Claims 1 to 4, characterized in that the peroxyacids used to prepare the clathrates are mixed with the corresponding organic acids in concentrations which may vary from a few percent to about 90% in moles, more particularly from about 10 to 40% and in that the peroxyacids which are solid at room temperature are in the form of mixtures in which the peroxyacid concentration is at least about 80% by weight.

6. Process for preparing peroxyacid derivatives according to Claim 1, characterized in that it comprises the addition of peroxyacid, pure or mixed with a compound, capable of acting as a receiving structure for the peroxyacid and recovery of the clathrate formed.

7. Process according to Claim 6, characterized in that peroxyacids which are solid at room temperature are advantageously dissolved in a suitable solvent - for example water or a mixture of water and a water soluble solvent such as an alcohol - and are added to the receiving compound, itself advantageously in solution, and in that the water soluble peroxyacids, notably alkylperoxyacids, for example those containing 1 to 6 carbon atoms, are in the form of mixtures with the corresponding carboxylic acids and hydrogen peroxide, a catalyst being added to these mixtures such as a strong mineral acid such as sulphuric acid or phosphoric acid or a sulphonic resin.

8. Process according to Claim 7, characterized in that the solution of peroxyacid contains a stabilizing agent such as dipicolinic acid.

9. Bleaching agents, characterized in that they contain at least one peroxyacid derivative according to any one of Claims 1 to 5, possibly in association with an inert vehicle.

10. Disinfectant agents, characterized in that they contain at least one peroxyacid derivative according to any one of Claims 1 to 5, possibly in association with an inert vehicle.

11. Cosmetic agents, characterized in that they contain at least one peroxyacid derivative according to any one of Claims 1 to 5, possibly in association with an inert vehicle.

12. Bodily hygiene agents, characterized in that they contain at least one peroxyacid derivative according to any one of Claims 1 to 5, possibly in association with an inert vehicle.

**13.** Medicaments including as the active ingredient at least one peroxyacid derivative according to any one of Claims 1 to 5.

**14.** Use of peroxyacid derivatives according to any one of Claims 1 to 5 for obtaining a medicament with antiseptic activity, particularly with a view to combating infections of the digestive tract, skin, mucous membranes or genital organs.

**15.** Agents or medicaments according to any one of Claims 9 to 14, characterized in that they are in the form of a powder, tablets or granules, possibly in association with an inert vehicle.

**16.** Use of a peroxyacid derivative according to any one of Claims 1 to 5 as a reagent in organic synthesis.

**Patentansprüche**

**1.** Peroxysäurederivate, dadurch gekennzeichnet, daß es sich um Einschlußverbindungen oder Clathrate handelt, die aus einer Peroxysäure bestehen, die in einem Hohlmolekül eingeschlossen ist, welches für die Peroxysäure die Rolle einer Aufnahmestruktur übernehmen kann.

**2.** Peroxysäurederivate nach Anspruch 1, dadurch gekennzeichnet, daß das Molekül, welches die Rolle einer Aufnahmestruktur übernimmt, die Struktur eines Cyclodextrins, insbesondere von Alpha-Cyclodextrin, Beta-Cyclodextrin oder von Gamma-Cyclodextrin, oder die von Cyclodextrinderivaten, hat.

**3.** Peroxysäurederivate nach Anspruch 1 oder 2, dadurch gekennzeichnet, daß man die Peroxysäure auswählt aus:

- Mono-Persäuren der Struktur der $R_1$-$CO_3H$,
  wobei $R_1$ eine Alkyl-, Aryl-, Cycloalkylgruppe darstellt,
- Diperoxysäuren der Struktur $HO_3C$-$R_2$-$CO_3H$,

wobei $R_2$ eine Alkylen-, Arylen-, Cycloalkylengruppe darstellt; wobei $R_1$ und $R_2$ bei Bedarf durch funktionelle Gruppierungen, insbesondere durch eine oder mehrere Carboxylfunktionen in Form eines Esters oder Amids, oder durch Salze, insbesondere Alkali-, Erdalkali-, Ammonium-, oder Phosphoniumsalze, und/oder durch eine oder mehrere Alkyl-, Alkoxy-, Arylgruppen, bei Bedarf mit einem oder mehreren Ringen, Amino-, Alkylamino-, Acylamino-, Acyl-, Nitril-, Nitro-, Trifluoromethyl-, Sulfonylgruppen und/oder ein oder mehrere Halogenatome substituiert sind.

**4.** Peroxysäurederivate nach Anspruch 3, dadurch gekennzeichnet, daß die Alkyl- oder Alkylenradikale 1 bis 12 Kohlenstoffatome enthalten, der Arylradikal ein Phenylradikal und der Arylenradikal ein Phenylenradikal ist.

**5.** Peroxysäurederivate nach einem der Ansprüche 1 - 4, dadurch gekennzeichnet, daß die zur Herstellung der Clathrate eingesetzten Peroxysäuren in Mischung mit den entsprechenden organischen Säuren mit Gehalten vorliegen, die zwischen einigen bis ungefähr 90 Mol-%, insbesondere von 10 bis ungefähr 40 %, schwanken können und daß die bei Umgebungstemperatur festen Peroxysäuren als Mischungen vorliegen, deren Gehalt an Peroxysäure mindestens ungefähr 80 Gew.-% ist.

**6.** Verfahren zur Darstellung von Peroxysäurederivaten nach Anspruch 1, dadurch gekennzeichnet, daß es die Hinzugabe reiner oder in Mischung vorliegender Peroxysäure zu einer Verbindung, die in der Lage ist, die Rolle einer Aufnahmestruktur für die Peroxysäure zu übernehmen, und die Wiedergewinnung des gebildeten Clathrats umfaßt.

**7.** Verfahren nach Anspruch 6, dadurch gekennzeichnet, daß die bei Umgebungstemperatur festen Peroxysäuren vorteilhafterweise in einem geeigneten Lösungsmittel in Lösung gebracht werden - z. B. Wasser oder eine Mischung Wasser/wasserlösliches Lösungsmittel, wie z. B. ein Alkohol - und zu der Aufnahmeverbindung, die ihrerseits vorteilhafterweise in Lösung vorliegt, hinzugefügt werden und die in wasserlöslichen Peroxysäuren, insbesondere Alkylperoxysäuren, wie z. B. solche, die 1 bis 6 Kohlenstoffatome enthalten, in Form von Mischungen mit den entsprechenden Carboxylsäuren und Wasserstoffperoxyd vorliegen, wobei diesen Mischungen ein Katalysator, wie z. B. eine starke Mineralsäure wie Schwefelsäure oder Phosphorsäure, oder ein Sulfonharz hinzugefügt wird.

8. Verfahren nach Anspruch 7, dadurch gekennzeichnet, daß die Peroxysäurelösung einen Stabilisator wie z. B. die Dipicolinsäure aufweist.

9. Bleichmittel, dadurch gekennzeichnet, daß sie mindestens ein Peroxysäurederivat nach einem der Ansprüche 1 bis 5, möglicherweise in Verbindung mit einem inerten Träger, enthalten.

10. Desinfektionsmittel, dadurch gekennzeichnet, daß sie mindestens ein Peroxysäurederivat nach einem der Ansprüche 1 bis 5, möglicherweise in Verbindung mit einem inerten Träger, enthalten.

11. Kosmetische Mittel, dadurch gekennzeichnet, daß sie mindestens ein Peroxysäurederivat nach einem der Ansprüche 1 bis 5, möglicherweise in Verbindung mit einem inerten Träger, enthalten.

12. Mittel zur Körperhygiene, dadurch gekennzeichnet, daß sie mindestens ein Peroxysäurederivat nach einem der Ansprüche 1 bis 5, möglicherweise in Verbindung mit einem inerten Träger, enthalten.

13. Medikamente, die als aktiven Wirkstoff mindestens ein Peroxysäurederivat nach einem der Ansprüche 1 bis 5 enthalten.

14. Verwendung der Peroxysäurederivate nach einem der Ansprüche 1 bis 5 für ein Medikament mit antiseptischer Wirkung, insbesondere zur Bekämpfung von Infektionen des Verdauungstraktes, der Haut, der Schleimhäute oder der Geschlechtsorgane.

15. Mittel oder Medikamente nach einem der Ansprüche 9 bis 14, dadurch gekennzeichnet, daß sie in Form von Pulver, Tabletten oder Granulaten, möglicherweise in Verbindung mit einem inerten Träger, vorliegen.

16. Verwendung eines Peroxysäurederivats nach einem der Ansprüche 1 bis 5 als Reagens in der organischen Synthese.